# DEMANDE DE BREVET EUROPEEN

(11) **EP 1 815 858 A2**
(43) Date de publication de la demande: **08.08.2007**
(21) Numéro de dépôt: 07075195.3
(22) Date de dépôt: 10.10.2003
(51) Int. Cl.: A61K 31/427, A61K 31/122, A61P 3/00

(54) **Association entre un ligand des récepteurs actives par les proliférateurs de péroxisomes et un agent antioxydant et les compositions pharmaceutiques qui les contiennent**

(30) Priorité: 11.10.2002 FR 0212646
(62) Demande divisionnaire de: 03807889.5
(71) Demandeur: Les Laboratoires Servier, 92415 Courbevoie Cedex (FR); Centre National de la Recherche Scientifique, 75794 Paris Cedex 16 (FR)
(72) Inventeur: Casteilla, Louis, 31750 Escalquens (FR); Penicaud, Luc, 31400 Toulouse (FR); Dacquet, Catherine, 59650 Villeneuve d'Asco (FR); Renard, Pierre, 78150 Le Chesnay (FR)
(74) Mandataire: Bestel, Delphine

(57) **Abrégé**

Association contenant un ou plusieurs ligands des récepteurs activés par les proliférateurs de peroxisomes et un agent antioxydant.

Médicaments.

## Description

La présente invention concerne l'association entre un ou plusieurs ligands sélectifs des récepteurs activés par les proliférateurs de peroxisomes (PPAR) et un agent antioxydant et son utilisation dans le traitement et/ou la prévention de l'obésité et des surcharges pondérales caractérisées par un index de poids corporel supérieur à 25.

L'obésité est un problème majeur de santé publique dans tous les pays développés. En progression constante également dans les pays en voie de développement, elle atteint une population de plus en plus jeune. L'obésité est un désordre chronique de déséquilibre énergétique caractérisé par un excès de prise énergétique à long terme comparé à une dépense énergétique limitée entraînant le stockage de cet excès d'énergie sous forme de tissu adipeux blanc.

Les surcharges en tissus adipeux contribuent directement à des problèmes de fatigue, d'essoufflement, d'apnée du sommeil et d'ostéoarthrite.

L'obésité est un facteur de risque bien établi pour le développement de l'insulino-résistance, de la dyslipidémie et en dernier lieu du diabète non insulino-dépendant. C'est un facteur favorisant les maladies cardiovasculaires et elle est associée à une augmentation significative des risques d'accidents vasculaires cérébraux, de calculs vésiculaires, de dysfonctions respiratoires, d'ostéoarthrite, de plusieurs formes de cancer et de mort prématurée.

La stratégie pharmacologique pour réduire l'obésité présente deux alternative : soit réduire la masse grasse en modifiant les entrées énergétiques et/ou en modifiant la répartition des nutriments entre la graisse et les tissus maigres, soit s'opposer ou réverser les conséquences métaboliques de l'augmentation de masse grasse sans nécessairement avoir un impact sur le degré d'obésité par lui-même.

Chez les obèses, il a été démontré que la génération d'espèces oxygénées réactives libérées par les monocytes et les leucocytes était fortement augmentée par rapport à des sujets non obèses (J. Clin. Endocrinol. Metab., 2001, 86, 355-362). Les concentrations plasmatiques élevées de facteur de nécrose tumorale alpha (TNFα) chez les obèses stimulent les processus inflammatoires (J. Clin. Endocrinol. Metab., 1998, 83, 2907-2910) et sont responsables de la génération d'espèces oxygénées réactives par les leucocytes (Oncogene, 1998, 17, 1639-1651).

L'état pathologique de l'obésité est également associé à une augmentation de l'oxydation des lipides et des protéines qui peut être à l'origine de concentrations plasmatiques importantes d'acides 9- et 13-hydroxyoctadécadiénoïques (9-HODE et 13-HODE) (Totowa : Humano. Press., 1998, 147-155), index clés de la peroxydation lipidique (J. Clin. Endocrinol. Metab., 2001, 86, 355-362). Parallèlement, les capacités "antioxydantes" de l'organisme diminuent.

Chez les sujets obèses, il a été démontré que la prise alimentaire excessive génère des dommages lipidiques et protéiques importants. La surconsommation de calories chez les obèses peut entraîner la génération de radicaux libres et les exposer à des lésions oxydatives importantes contribuant à maintenir l'état d'obésité.
Les marqueurs spécifiques de l'oxydation sont significativement diminués lors d'une diète de 48 heures ou d'une restriction calorique qui s'accompagne de perte de poids (J. Clin. Endocrinol. Metab., 2001, 86, 355-362).

Une stratégie visant à réduire la "charge en oxydation" de l'organisme tout en favorisant les métabolismes lipidique et glucidique devrait conduire à une exacerbation des effets et par voie de conséquence à une perte de poids chez les sujets obèses ou présentant une surcharge pondérale.

Parmi les composés capables de favoriser les métabolismes lipidique et glucidique, les ligands sélectifs des récepteurs activés par les proliférateurs de peroxisome ou PPAR sont des composés particulièrement intéressants.

Les PPAR(s) sont une famille de récepteurs nucléaires hormonaux comprenant trois sous-types distincts : α, β (encore appelé δ ou NUC1) et γ (qui présente trois isoformes : γ₁, γ₂ et γ₃).

Ils ont été initialement clonés comme des récepteurs nucléaires qui médient les effets de proliférateurs de peroxisomes sur la transcription génique, mais il est clair qu'un très grand nombre de composés naturels comme les eicosanoïdes et les acides gras peuvent également activer les PPAR(s).

Comme un certain nombre d'autres récepteurs nucléaires aux hormones, les protéines PPAR(s) se lient sur des promoteurs sous la forme d'hétérodimères avec le récepteur à l'acide 9 cis rétinoïque : RXR. L'hétérodimère PPAR/RXR peut être activé par la liaison d'un ligand spécifique de l'un des deux récepteurs mais l'activation maximale s'effectue avec la présence de deux ligands.

Les PPAR(s) sont des facteurs de transcription, dépendants des ligands, ce qui signifie que l'activation de la transcription des gènes cibles dépend strictement de la liaison du ligand.

Certains ligands comme les acides gras mono ou polyinsaturés ou les acides gras saturés se lient sur les trois sous-types de récepteurs. Les acides gras polyinsaturés à longue chaîne comme l'acide linolénique ou des acides gras conjugués ou oxydés se lient avec une haute affinité sur PPARα.

La plus importante fonction des PPAR(s) provient de leur expression tissu-dépendante et de l'identité de leurs gènes cibles très souvent impliqués exclusivement dans le transport et le métabolisme des lipides et des carbohydrates.

La souris PPARα KO développe de l'obésité et de l'hypertriglycéridémie même si la prise calorique quotidienne n'augmente pas. Ces effets sont expliqués en grande partie par la réduction de la capture des acides gras par le foie et l'inhibition de leur oxydation (J. Biol. Chem., 1998, 273, 29577-29585).

Le foie est un organe à capacité oxydative pour les acides gras. Lorsque l'oxydation hépatique des acides gras est optimisée, la thermogénèse s'enclenche et transforme l'énergie disponible en chaleur avec diminution du quotient respiratoire et augmentation de la vitesse du métabolisme basal. Ces circonstances sont très favorables à la perte de tissu adipeux (Med. Hypotheses, 1999, 52(5), 407-416).

La stratégie consistant à désinhiber les processus enzymatiques de l'oxydation hépatique des acides gras tout en assurant une stimulation transcriptionnelle des gènes activés par les PPAR(s) et impliqués dans les processus métaboliques lipidique et glucidique doit aboutir à une diminution des acides gras libres plasmatiques et une lipolyse modérée des adipocytes constituant le tissu adipeux viscéral entraînant à long terme une régression de l'obésité viscérale et donc une perte de poids corporel.

La présente invention concerne plus particulièrement l'association entre un ou plusieurs composés ligands des récepteurs activés par les proliférateurs de peroxisomes et un agent antioxydant.

Cette association présente des propriétés pharmacologiques tout à fait remarquables dans le domaine de l'obésité.

Plus particulièrement, les ligands PPAR selon l'invention sont des ligands sélectifs des sous-types réceptoriels α et/ou γ.

Avantageusement, l'association selon l'invention contient un ligand sélectif PPARα et un ligand sélectif PPARγ.

Une variante avantageuse concerne l'association selon l'invention dans laquelle le ligand PPAR est un ligand mixte des sous-types réceptoriels α et γ.

Les ligands PPARα et/ou γ selon l'invention sont avantageusement représentés par le gemfibrozyl, le WY-14,643, la pioglitazone et encore plus préférentiellement par la rosiglitazone.

Les ligands PPAR selon l'invention sont également représentés par les composés décrits dans les demandes WO 9736579, WO 9731907, WO9728115, WO9638415, WO9727857, WO9725042, WO9701420, WO9640128, WO2000064888 et WO2000064876.

Les agents antioxydants selon l'invention sont représentés par différentes catégories de composés :
- des agents antiradicalaires ou piégeurs de radicaux libres,
- des agents antilipoperoxydants,
- des agents chélatants,
- des agents capables de régénérer les antioxydants endogènes comme le glutathion, la vitamine C ou la vitamine E.

L'agent antioxydant de l'association selon l'invention est plus préférentiellement représenté par des dérivés de quinones comme l'ubiquinone ou coenzyme Q₁₀, qui agit en tant que piégeur de radicaux libres mais qui est également capable de régénérer de la vitamine E.

Les énantiomères, diastéréoisomères ainsi que les sels d'addition à un acide ou à une base pharmaceutiquement acceptable des composés ligands PPAR et antioxydants selon l'association font également partie intégrante de l'invention.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la *tert*-butylamine, etc...

L'association préférée selon l'invention est la rosiglitazone et le coenzyme Q₁₀.

Par ailleurs, l'association selon l'invention entre un ou plusieurs composé favorisant les métabolismes lipidique et glucidique et un agent antioxydant possède des propriétés pharmacologiques tout à fait surprenantes : la demanderesse a en effet mis en évidence l'existence d'une synergie entre ces deux classes de composés permettant d'obtenir une réduction très significative de la masse grasse corporelle la rendant utile dans le traitement et/ou la prévention de l'obésité et des surcharges pondérales caractérisées par un index de poids corporel supérieur à 25.

L'obésité aux Etats-Unis atteint 20 % des hommes et 25 % des femmes. Sont considérés comme obèses les patients d'indice de poids corporel (IMC = poids (kg) / taille² (m²)) supérieur ou égal à 30 (Int. J. Obes., 1998, 22, 39-47 ; Obesity Lancet, 1997, 350, 423-426). L'obésité (IMC ≥ 30) et les surcharges pondérales (25 < IMC < 30) peuvent avoir plusieurs origines : elles peuvent survenir à la suite d'une dérégulation de la prise de nourriture, d'une dérégulation hormonale ou encore à la suite de l'administration d'un traitement : un traitement antidiabétique de type II avec les sulphonylurées entraîne une prise de poids chez les patients. De même dans le diabète de type I (insulino-dépendant), l'insulinothérapie est également une source de prise de poids corporel chez les malades (In Progress in Obesity Research, 8th International Congress on Obesity, 1999, 739-746; Annals of Internal Medicine, 1998, 128, 165-175).

L'obésité et les surcharges pondérales sont des facteurs de risque bien établis pour les maladies cardiovasculaires : elles sont associées à une augmentation significative des risques d'accidents vasculaires cérébraux, de diabète non-insulino-dépendant car elles prédisposent à l'insulino-résistance, aux dyslipidémies et à l'apparition de maladies macrovasculaires (néphropathies, rétinopathies, angiopathies).
D'autres pathologies sont la conséquence de l'obésité ou de surcharges pondérales : on peut citer en particulier les calculs vésiculaires, les dysfonctions respiratoires, l'ostéoarthrite, plusieurs formes de cancers et dans les cas d'obésité très sévère la mort prématurée (N. Engl. J. Med., 1995, 333, 677-385 ; JAMA, 1993, 270, 2207-2212).
L'association selon l'invention permet d'obtenir une perte de poids qui même modérée réduit significativement tous les facteurs de risque associés à l'obésité (Int. J. Obes., 1997, 21, 55-9 ; Int. J. Obes., 1992, 21, S5-9).

L'association selon l'invention trouve donc son utilité dans le traitement et/ou la prévention de l'obésité et des surcharges pondérales caractérisées par un index de poids corporel supérieur à 25 et inférieur à 30.
L'invention concerne donc l'utilisation d'une association contenant un ou plusieurs ligands PPAR et un agent antioxydant pour l'obtention de compositions pharmaceutiques destinées au traitement et/ou à la prévention de l'obésité ainsi que des surcharges pondérales caractérisées par un index de poids corporel supérieur à 25 et inférieur à 30.

En particulier, l'association selon l'invention est utile dans le traitement et/ou la prévention de l'obésité et des surcharges pondérales caractérisées par un index de poids corporel supérieur à 25 et inférieur à 30 induites par un traitement thérapeutique, comme le traitement du diabète de type I ou II.
L'invention concerne donc l'utilisation d'une association contenant un ou plusieurs ligands PPAR et un agent antioxydant pour l'obtention de compositions pharmaceutiques destinées au traitement et/ou à la prévention de l'obésité ainsi que des surcharges pondérales caractérisées par un index de poids corporel supérieur à 25 et inférieur à 30 induites par un traitement thérapeutique, comme le traitement du diabète de type I ou II.

L'invention concerne également les compositions pharmaceutiques contenant l'association entre un ou plusieurs dérivés favorisants les métabolismes lipidique et glucidique et plus particulièrement un ou plusieurs ligands PPAR et un agent antioxydant telle que définie précédemment en combinaison avec un ou plusieurs excipients pharmaceutiquement acceptable.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie varie selon le sexe, l'âge et le poids du patient, la voie d'administration, la nature de l'indication thérapeutique ou des traitements éventuellement associés et s'échelonne entre 0,1 mg et 1 g de chaque composant de l'association par 24 heures en une ou plusieurs prises.

Les exemples suivants illustrent l'invention mais ne la limitent en aucune façon.

### EXEMPLE A : Variation du poids corporel

Des souris mâles C57 Black 6 ob/ob de 8 à 12 semaines ont été utilisées. Ces souris son diabétiques (type II) et souffrent d'hyperglycémie, d'hypertriglycéridémie et d'hyperinsulinémie. Après mise en quarantaine d'une semaine, elles ont été pesées puis randomisées en fonction de leur poids, et 6 groupes homogènes (poids de départ non significativement différent) ont été formés. Après avoir été pesées, ces souris sont traitées à la rosiglitazone (antidiabétique) seule ou en association avec le coenzyme Q₁₀. Les molécules sont injectées par voie intrapéritonéale une fois par jour pendant 14 jours dans une solution DMSO 5 % / Solutol 15 % / Qsp H₂O chauffée à 65°C pour assurer une bonne dissolution. La solution est de plus préchauffée avant injection. Les souris sont pesées tous les jours et le poids obtenu après 14 jours de traitement est relevé.
Le traitement avec la rosiglitazone seule conduit à une augmentation de poids des souris supérieure ou égale à 5 grammes correspondant environ à 10% de plus que leur poids initial. L'association rosiglitazone+coenzyme Q₁₀ permet de réverser cette prise de poids d'au moins 50% et met en évidence l'efficacité de l'association dans la réduction du poids corporel.

### EXEMPLE B : Composition pharmaceutique

### 100 comprimés dosés à 30 mg de rosiglitazone et 10 mg de coenzyme Q₁₀

| | |
|---|---|
| Rosiglitazone | 3 g |
| Coenzyme Q₁₀ | 1 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Association contenant la rosiglitazone ou un de ses sels d'addition à un acide ou à une base pharmaceutiquement acceptable et le coenzyme Q₁₀.

2. Compositions pharmaceutiques contenant la rosiglitazone ou un de ses sels d'addition à un acide ou à une base pharmaceutiquement acceptable et le coenzyme Q₁₀ comme principe actif selon la revendication 1 seuls ou en combinaison avec un ou plusieurs excipients pharmaceutiquement acceptables.

3. Compositions pharmaceutiques selon la revendication 2 utiles pour la fabrication d'un médicament pour le traitement et/ou la prévention de l'obésité.

4. Compositions pharmaceutiques selon la revendication 2 utiles pour la fabrication d'un médicament pour le traitement et/ou la prévention de l'obésité induite par un traitement thérapeutique.

5. Compositions pharmaceutiques selon la revendication 2 utiles pour la fabrication d'un médicament pour le traitement et/ou la prévention de l'obésité induite par un traitement du diabète de type I ou II.

6. Compositions pharmaceutiques selon la revendication 2 utiles pour la fabrication d'un médicament pour le traitement et/ou la prévention des surcharges pondérales **caractérisées par** un index de poids corporel supérieur à 25 et inférieur à 30.

7. Compositions pharmaceutiques selon la revendication 2 utiles pour la fabrication d'un médicament pour le traitement et/ou la prévention des surcharges pondérales **caractérisées par** un index de poids corporel supérieur à 25 et inférieur à 30 induites par un traitement thérapeutique.

8. Compositions pharmaceutiques selon la revendication 2 utiles pour la fabrication d'un médicament pour le traitement et/ou la prévention des surcharges pondérales **caractérisées par** un index de poids corporel supérieur à 25 et inférieur à 30 induites par un traitement du diabète de type I ou II.

9. Utilisation d'une association selon la revendication 1 pour l'obtention de compositions pharmaceutiques destinées au traitement et/ou à la prévention de l'obésité.

10. Utilisation d'une association selon la revendication 1 pour l'obtention de compositions pharmaceutiques destinées au traitement et/ou à la prévention de l'obésité induite par un traitement thérapeutique.

11. Utilisation d'une association selon la revendication 1 pour l'obtention de compositions pharmaceutiques destinées au traitement et/ou à la prévention de l'obésité induite par un traitement du diabète de type I ou II.

12. Utilisation d'une association selon la revendication 1 pour l'obtention de compositions pharmaceutiques destinées au traitement et/ou à la prévention des surcharges pondérales **caractérisées par** un index de poids corporel supérieur à 25 et inférieur à 30.

13. Utilisation d'une association selon la revendication 1 pour l'obtention de compositions pharmaceutiques destinées au traitement et/ou à la prévention des surcharges pondérales **caractérisées par** un index de poids corporel supérieur à 25 et inférieur à 30 induites par un traitement thérapeutique.

14. Utilisation d'une association selon la revendication 1 pour l'obtention de compositions pharmaceutiques destinées au traitement et/ou à la prévention des surcharges pondérales **caractérisées par** un index de poids corporel supérieur à 25 et inférieur à 30 induites par un traitement du diabète de type I ou II.
